# EUROPEAN PATENT APPLICATION

(11) **EP 1 208 807 A1**
(43) Date of publication of application: **29.05.2002**
(21) Application number: 99952825.0
(22) Date of filing: 06.08.1999
(51) Int. Cl.: A61B 18/08

(54) **DEVICE FOR COAGULATION OF TISSUES**

(71) Applicant: Intellikraft Limited, London, EC1M 4JN (GB)
(72) Inventor: SPIRIN, Jury Leonidovich, Moscow, 117261 (RU); KRUTOVA, Elena Igorevna, Moscow, 117261 (RU); DUBININ, Vladimir Stepanovich, Moscow, 119048 (RU); FROLOV, Dmitry Vladimirovich, Moscow, 117574 (RU)
(74) Representative: Harrison Goddard Foote
(86) International application number: RU9900276
(87) International publication number: WO0110318

(57) **Abstract**

A device for the electrical coagulation of tissues, containing a high voltage supply source, an effective power regulator and a probe, is provided with a piezo-electric voltage transformer, thanks to which automatic regulation of effective power in the process of coagulation and automatic switching off of the device when the coagulation process is complete are implemented, which enables the depth of necrosis of the tissues to be reduced.

## Description

### Technology field.

The invention relates to the field of medicine and is intended for the coagulation of tissues during operational intervention in hospital and medical centre conditions.

### Prior art.

There is a known laser tissue coagulator (see B. Pyer, Equipment for intracavitary operations using a laser and an electric knife, Moscow, Meditsina, 1996, pp. 77-78), consisting of a series-wired electro-optical amplifier and probe, in which the controlling input of the electro-optical amplifier is connected to the effective power control input. The effective power is altered in accordance with preset parameters.

The problem with this coagulator is the quite broad necrosis, due to the absence of control of the effective power of the electro-optical converter when the depth of the necrosis is increased in conditions in which the coagulation process is completed.

There is a known electrical tissue coagulator (see B. Pyer, Equipment for intracavitary operations using a laser and an electric knife, Moscow, Meditsina, 1996, p. 115), consisting of a series-wired high-voltage electricity supply source, effective power regulator and probe, in which the controlling input of the amplifier is connected to the effective power control input.

As for the previous coagulator, the effective power is altered in accordance with preset values, as a result of which deep necrosis is possible, due to the absence of control of the effective power of the electro-optical converter when the depth of the necrosis is increased in conditions in which the coagulation process is completed.

### Substance of the invention.

The aim of this invention is to create a tissue coagulator giving reduced depth of necrosis of the tissues.

This problem is solved by the invention due to the creation of a coagulator with automatic regulation of the effective power. This has been made possible by the introduction into the device of a piezo-electric voltage transformer, the power input of which is connected to the power regulator output, and the controlling input to a direct current supply, with the output connected to the probe. Control of the power applied to the tissues proceeds automatically due to the shift of the resonance frequency of the piezo-electric transformer towards smaller or greater values. At the initial stage of operation of the coagulator, the resonance frequency is shifted "to the left", towards the smaller values, by 0.5-0.6%. This fact makes it possible to reduce the effective power in the initial period of time in order to prevent or at least reduce the influence of trembling of the surgeon's hands on the operation of the device. In the process of coagulation, at the stage of the bonding of the vessels, the resistance of the tissue increases, leading to a shift "to the right" of the resonance frequency, towards the greater values, as a result of which the effective power increases. When necrosis effects appear, the resistance of the tissue decreases sharply, leading to a sharp shift "to the right" of the resonance frequency of the piezo-electric transformer, as a result of which voltage is automatically taken off from the probe.

### Brief description of drawings

Figure 1 shows the structural layout of the tissue coagulation device in accordance with this invention.
Figure 2 shows the change in the frequency characteristics of the piezo-electric voltage transformer in the course of the operation of the device.

### Best variant for implementation of the invention.

The device contains a high voltage electricity supply source 1, power regulator 2, piezo-electric voltage transformer 3 and probe 4.

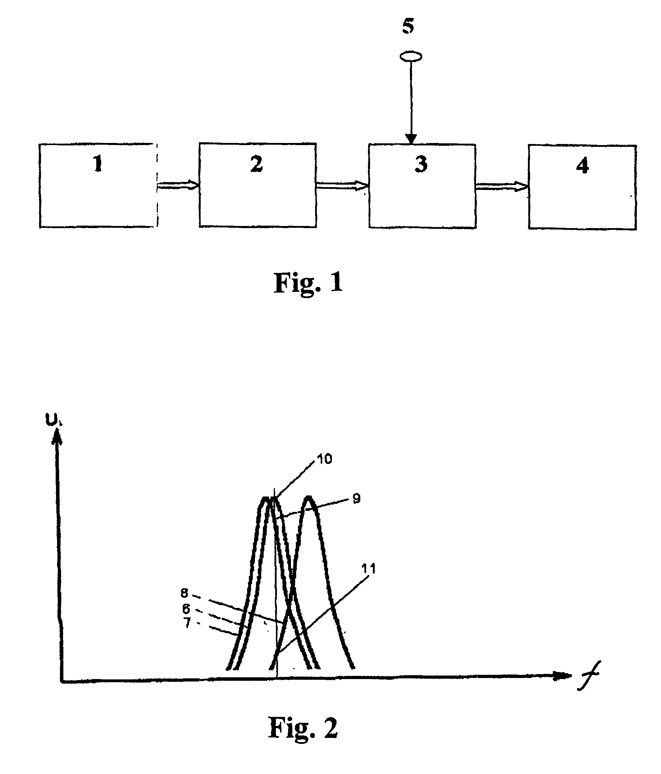

The sine curve signal of amplitude 300-500 V, the frequency of which corresponds to the resonance frequency of the piezo-electric voltage transformer 3 (graph 6, point 10 on fig. 2), goes from the input of the high voltage electricity supply source I to the input of the effective power regulator 2 (the power is selected on the basis of the type of operation and is of a magnitude of 10-60 mW), after which the signal goes to the input of the piezo-electric voltage transformer 3, where it is amplified in voltage to a magnitude of 1-5 kW At the initial moment, voltage of 100-120 V is supplied to the controlling input of the piezo-electric transformer 3 from the constant voltage busbar 5. This displaces the resonance frequency f of the piezo-electric transformer to the left by 0.5-0.6% (graph 7 on fig. 2), as a result of which the effective power (voltage U at the output of the piezo-electric transformer) is somewhat reduced (point 9 on fig. 2). In the process of coagulation at the vessel bonding stage, the equivalent resistance of the tissue at the output of the probe 4 grows. This leads to an increase in the effective power (point 10 on fig. 2) due to the shift of the resonance frequency to the right (graph 6 on fig. 2). When necrosis effects appear, accompanied by a sharp reduction in the resistance of the tissue, the equivalent resistance of the tissue falls sharply and the resonance frequency of the piezo-electric voltage transformer shifts sharply to the right (graph 8 on fig. 2), thus reducing the voltage to a value corresponding to point 11 on graph 8.

The device applied for makes it possible sharply to reduce the depth of necrosis by comparison with the known tissue coagulators.

Thus, for liver operations, the depth of necrosis is reduced from 50-40 µm for the known devices to 10 µm for the device applied for, with improved quality of coagulation.

For operations on the tibia, the depth of necrosis is reduced from 200-150 µm to 20 µm with improved quality of coagulation.

## Claims

1. Device for the coagulation of tissues, containing, wired in series, a high voltage electricity supply source, an effective power regulator and a probe, ***characterised* in that** it has a piezo-electric voltage transformer, the power input of which is connected to the said effective power regulator and the controlling input to a constant voltage source, while the output is connected to the probe.

2. Process for the electrical coagulation of tissues using the device described in claim 1, in which at the beginning of the coagulation process, the resonance frequency of the piezo-electric transformer is displaced towards the side of lower frequency values, thus reducing the effective power by an extent sufficient to exclude the influence of trembling of the surgeon's hands on the operation of the device.

3. Process in accordance with claim *2,* ***characterised* in that** the displacement of the resonance frequency is implemented to a magnitude of 0.5-0.6%.
